# EUROPÄISCHE PATENTSCHRIFT

(11) **EP 4 702 954 B1**
(45) Veröffentlichungstag und Bekanntmachung des Hinweises auf die Patenterteilung: **11.11.2026**
(21) Anmeldenummer: 24196698.5
(22) Anmeldetag: 27.08.2024
(51) Int. Cl.: A61F 13/42, A61F 13/532

(54) **HYGIENEARTIKEL**
SANITARY ARTICLE
ARTICLE D'HYGIÈNE PERSONNELLE

(43) Veröffentlichungstag der Anmeldung: 04.03.2026
(73) Patentinhaber: LK Mahnke GmbH & Co. KG, 45479 Mülheim an der Ruhr (DE)
(72) Erfinder: PAZ, Rui Miguel, 47800 Krefeld (DE)
(74) Vertreter: Gottschald Patentanwälte Partnerschaft mbB

(56) Entgegenhaltungen:
- WO-A1-2014/073636
- US-A1- 2006 129 116
- US-A1- 2023 143 877
- US-B2- 11 191 679
- US-B2- 8 388 590

## Beschreibung

Die vorliegende Erfindung betrifft einen Hygieneartikel gemäß dem Oberbegriff von Anspruch 1 sowie ein Verfahren zur Herstellung eines Hygieneartikels gemäß dem Oberbegriff von Anspruch 14.

Der Begriff "Hygieneartikel" im Zusammenhang mit dieser Anmeldung ist breit zu verstehen und umfasst insbesondere Windeln, wie beispielsweise Babywindeln, aber auch Erwachsenenwindeln und Inkontinenzwindeln, sowie Binden, Pants und Hygienevorlagen.

Ganz grundsätzlich weist der in Rede stehende Hygieneartikel einen absorbierenden Kern auf. Der absorbierende Kern ermöglicht die Aufnahme von Flüssigkeit, welche etwa von einem Träger des Hygieneartikels abgegeben wird, beispielsweise Urin. Der absorbierende Kern weist ein körperseitiges Deckblatt, ein körperfernes Rückenblatt und eine zwischen dem Deckblatt und dem Rückenblatt angeordnete Schicht mit Saugmaterial auf. Das körperseitige Deckblatt ist während der Verwendung des Hygieneartikels dem Träger zugewandt, das körperferne Rückenblatt ist während der Verwendung des Hygieneartikels dem Träger abgewandt. Die Schicht mit Saugmaterial ermöglicht es, dass vergleichsweise viel Flüssigkeit von dem absorbierenden Kern aufgenommen werden kann. Um eine gute Flüssigkeitsverteilung innerhalb des absorbierenden Kerns zu erreichen, weist dieser neben dem Deckblatt, dem Rückenblatt und der Schicht mit Saugmaterial einen Längskanal auf. Der Längskanal verläuft im Wesentlichen mittig entlang der Längsachse und unterteilt die Schicht mit Saugmaterial in zwei Saugabschnitte. Der in Rede stehende Hygieneartikel weist ferner einen Indikator auf. Der Indikator ermöglicht die Anzeige von durch den absorbierenden Kern aufgenommener Flüssigkeit. Durch den Indikator lässt sich durch eine vergleichsweise wenig aufwändige Sichtprüfung erkennen, ob der absorbierende Kern bereits Flüssigkeit aufgenommen hat und ob der Hygieneartikel ggf. gewechselt werden sollte.

Der bekannte Stand der Technik (DE 20 2022 002 784 U1), von dem die Erfindung ausgeht, betrifft einen solchen Hygieneartikel. Der Indikator umfasst bei dem bekannten Hygieneartikel einen Indikatorstreifen, welcher mittig entlang der Längsachse des absorbierenden Kerns verläuft. Hierbei ist vorgesehen, dass der Indikatorstreifen zum Teil mittig an dem Längskanal selbst und zum Teil mittig entlang der Längsachse an der Schicht mit Saugmaterial verläuft.

Obschon bei dem bekannten Hygieneartikel der Indikator mit einem Indikatorstreifen bereits ermöglicht, zu erkennen, inwieweit Flüssigkeit von dem absorbierenden Kern aufgenommen wurde, gibt es hier gewisse Herausforderungen hinsichtlich der Anzeigezuverlässigkeit. Insbesondere ist es bei dem bekannten Hygieneartikel möglich, dass ein Teil des Indikatorstreifens vergleichsweise früh und ein anderer Teil des Indikatorstreifens vergleichsweise spät die Flüssigkeitsaufnahme anzeigt. WO2014073636 und US2023143877 stellen weitere bekannte Stand der Technik Dokumente dar.

Der Erfindung liegt vor diesem Hintergrund das Problem zugrunde, den bekannten Hygieneartikel derart auszugestalten und weiterzubilden, dass hinsichtlich der genannten Herausforderung eine Optimierung erreicht wird, insbesondere wird die Anzeigezuverlässigkeit des Indikators verbessert.

Das obige Problem wird durch die Merkmale von Anspruch 1 gelöst.

Wesentlich ist die grundsätzliche Überlegung, dass der Indikator zwei Indikatorstreifen umfasst, welche bezogen auf den Längskanal jeweils auf unterschiedlichen Seiten angeordnet und entsprechend dem jeweiligen der Saugabschnitte zugeordnet sind. Im Stand der Technik weist der Indikator einen mittig entlang des Längskanals angeordneten Indikatorstreifen auf. Sobald hier Flüssigkeit über den Längskanal verteilt wird, wird diese durch den Indikatorstreifen angezeigt - weitestgehend ganz unabhängig davon, ob überhaupt eine gewisse Menge an Flüssigkeit von der Schicht mit absorbierendem Material aufgenommen ist oder nicht. Eine derartig verfrühte Anzeige durch die Indikatorstreifen kann vorschlagsgemäß vermieden werden, indem die Indikatorstreifen zumindest abschnittsweise in den Saugabschnitten, links- und rechtsseitig von dem Längskanal, angeordnet sind. Die Indikatorstreifen werden also eben genau dort angeordnet, wo die Flüssigkeit absorbiert ist bzw. wird, und nicht bloß dort, wo die Flüssigkeit im getragenen Zustand in der Regel vergleichsweise früh, ggf. zuerst, auftritt (nämlich im Bereich der Längsachse) oder verteilt wird (nämlich im Längskanal). Um zu erreichen, dass die Anzeigezuverlässigkeit verbessert ist, ist eine gute Flüssigkeitsverteilung in dem absorbierenden Kern erforderlich. Dies erfolgt maßgeblich durch den im Wesentlichen mittig entlang der Längsachse des absorbierenden Kerns verlaufenden Längskanal. Ohne den Längskanal könnte es ggf. zu einer verspäteten Anzeige durch den Indikator kommen. Durch die vorschlagsgemäße Ausgestaltung kann damit eine verfrühte, aber auch eine verspätete Anzeige durch den Indikator mit Indikatorstreifen vermieden werden. Durch die Anordnung der Indikatorstreifen auf unterschiedlichen Seiten des Längskanals kann sich auch eine verbesserte Anzeigezuverlässigkeit ergeben, wenn Flüssigkeit primär über einen der Saugabschnitte des Hygieneartikel aufgenommen wird. Dies ist beispielsweise möglich, wenn der Träger auf einer Körperseite liegt und die Flüssigkeit im Bereich dieses Saugabschnittes eingeleitet wird. Zumindest einer der Indikatorstreifen kann sodann die Aufnahme der Flüssigkeit rechtzeitig anzeigen.

Im Einzelnen wird vorgeschlagen, dass der Indikator zwei Indikatorstreifen umfasst und dass einer der Indikatorstreifen linksseitig und einer der Indikatorstreifen rechtsseitig von dem Längskanal angeordnet und dem jeweiligen der Saugabschnitte zugeordnet ist.

Die Ansprüche 2 und 3 betreffen vorteilhafte Ausgestaltungen, welche eine verbesserte Flüssigkeitsverteilung im absorbierenden Kern ermöglichen. Durch Verteilerkanäle gemäß Anspruch 2 lässt sich herstellungstechnisch einfach, doch gleichermaßen funktional Flüssigkeit in dem absorbierenden Kern, beispielsweise in die Saugabschnitte, verteilen. Die Flüssigkeit kann durch die Verteilerkanäle insbesondere aus der Mitte des absorbierenden Kerns, etwa ausgehend von dem Längskanal, hin zu Bereichen des absorbierenden Kerns, welche nicht unmittelbar an den Längskanal angrenzen, verteilt werden. Umgekehrt kann ebenso Flüssigkeit durch die Verteilerkanäle von den Bereichen des absorbierenden Kerns, welche nicht unmittelbar an den Längskanal angrenzen, zu der Mitte des absorbierenden Kerns hin, etwa hin zu dem Längskanal, verteilt werden. Die Verteilung durch die Verteilerkanäle kann dabei grundsätzlich in Richtung der Längsachse und/oder in Richtung der Querachse des absorbierenden Kerns erfolgen. Eine hinsichtlich der Anatomie des Trägers angepasste Flüssigkeitsverteilung lässt sich gemäß Anspruch 3 realisieren.

Die Ansprüche 4 bis 9 sind auf vorteilhafte Ausgestaltungen gerichtet, welche die Indikatorstreifen betreffen.

Sowohl der Längskanal als auch die Verteilerkanäle ermöglichen die Flüssigkeitsverteilung innerhalb des absorbierenden Kerns, weshalb in den Kanälen vergleichsweise frühzeitig Flüssigkeit sein kann. Um eine verfrühte Anzeige durch die Indikatorstreifen zu vermeiden, können diese vorteilhaft schnittpunktfrei gegenüber dem Längskanal und/oder den Verteilerkanälen angeordnet sein (Anspruch 4).

Gleichwohl mag es auch Anwendungsfälle geben, bei welchen eine vergleichsweise frühzeitige Anzeige durch die Indikatorstreifen erforderlich ist, beispielsweise bei Hygieneartikel für empfindliche Haut des Trägers. Aus diesem Grund sieht eine vorteilhafte Ausgestaltung vor, dass die Indikatorstreifen und der Längskanal und/oder die Verteilerkanäle jeweils zumindest einen gemeinsamen Schnittpunkt haben (Anspruch 5).

Eine symmetrische Anordnung (Anspruch 6) der Indikatorstreifen gegenüber der Längsachse kann die Anzeigezuverlässigkeit weiter verbessern. Dies kann insbesondere bei einem symmetrischen Aufbau des Hygieneartikels und/oder des absorbierenden Kerns, welcher bzw. welche zu einer gleichmäßigen Flüssigkeitsverteilung führen kann bzw. können, gegeben sein.

Einfach und gleichermaßen funktional können die Indikatorstreifen vorteilhaft jeweils als zusammenhängende Indikatorstreifen, also ohne etwaige Unterbrechungen entlang des jeweiligen Indikatorstreifens, ausgebildet sein (Anspruch 7). Dies kann insbesondere bei einem symmetrischen Aufbau des Hygieneartikels und/oder des absorbierenden Kerns, welcher bzw. welche zu einer gleichmäßigen Flüssigkeitsverteilung führen kann bzw. können, vorteilhaft sein. Auch aus herstellungstechnischer Sicht kann dies zu Vorteilen führen, etwa da das Anordnen der Indikatorstreifen vereinfacht wird. Doch auch das Vorsehen von einer oder mehreren Unterbrechungsstellen (Anspruch 7) kann die Anzeigezuverlässigkeit verbessern, indem etwa bestimmte Bereiche, in welchen eine ungewöhnlich hohe oder geringe Flüssigkeitsaufnahme zu erwarten ist, gezielt indikatortechnisch ausgelassen werden können.

Gemäß Anspruch 8 können die Indikatorstreifen jeweils einen geraden Indikatorabschnitt und/oder einen gekrümmten Indikatorabschnitt aufweisen. Insbesondere der gerade Indikatorabschnitt kann fertigungstechnisch einfach umzusetzen sein. Insbesondere der gekrümmte Indikatorabschnitt kann die Anzeigezuverlässigkeit verbessern, indem beispielsweise die Flüssigkeitsaufnahme in zu dem Längskanal unterschiedlich weit entfernten Bereichen des absorbierenden Kerns anzeigbar ist.

Eine weitere vorteilhafte Ausgestaltung gemäß Anspruch 9 sieht vor, dass sich die Indikatorstreifen entlang der Längsachse des absorbierenden Kerns über eine gewisse Mindestlänge des absorbierenden Kerns und/oder des Längskanals erstrecken. Hierdurch lässt sich die Anzeigezuverlässigkeit weiter verbessern.

In Anspruch 10 ist eine weitere vorteilhafte Ausgestaltung beansprucht, welche die Anordnung der Indikatorstreifen an einem Backsheet vorsieht. Hierdurch lässt sich erreichen, dass die Indikatorstreifen, insbesondere im getragenen Zustand des Hygieneartikels, von außen erkennbar sind.

Der Längskanal lässt sich herstellungstechnisch einfach, doch gleichermaßen funktional zuverlässig durch die vorteilhafte Ausgestaltung gemäß Anspruch 11 ausbilden.

Durch das Vorsehen von Faltungsbahnen (Anspruch 12) lässt sich in einer weiteren vorteilhaften Ausgestaltung der Tragekomfort weiter verbessern.

Eine weitere vorteilhafte Ausgestaltung gemäß Anspruch 13 sieht vor, dass die Indikatorstreifen zumindest abschnittsweise entlang der Faltungsbahnen verlaufen. Hierdurch kann die Sichtbarkeit der Indikatorstreifen verbessert werden, beispielsweise indem die Indikatorstreifen auf jenen zueinander schräg orientierten Faltflächen liegen, welche sich durch das Falten des Hygieneartikels entlang der Faltungsbahnen ergeben, und damit aus unterschiedlichen Winkeln erkennbar sind.

Nach einer weiteren Lehre gemäß Anspruch 14, der eigenständige Bedeutung zukommt, wird ein Verfahren zur Herstellung eines, vorschlagsgemäßen, Hygieneartikels, insbesondere einer Windel, beansprucht.

Dabei ist wesentlich, dass der Indikator zwei Indikatorstreifen umfasst und dass einer der Indikatorstreifen linksseitig und einer der Indikatorstreifen rechtsseitig von dem Längskanal angeordnet und dem jeweiligen der Saugabschnitte zugeordnet wird.

Auf alle Ausführungen zu dem vorschlagsgemäßen Hygieneartikel darf verwiesen werden.

Im Folgenden wird die Erfindung anhand einer lediglich Ausführungsbeispiele darstellenden Zeichnung näher erläutert. In der Zeichnung zeigt
- Fig. 1: einen vorschlagsgemäßen Hygieneartikel in perspektivischer Ansicht,
- Fig. 2: in a) den Hygieneartikel gemäß Fig. 1 in einem getragenen Zustand an einem Träger, in b) den Hygieneartikel gemäß Fig. 1 in weiterer perspektivischen Ansicht, und in c) eine weitere Ausführungsform des Hygieneartikels in perspektivischer Ansicht,
- Fig. 3: den Hygieneartikel gemäß Fig. 1 in Explosionsdarstellung,
- Fig. 4: in a) bis f) unterschiedliche Ausführungsbeispiele eines absorbierenden Kerns und eines Indikators mit Indikatorstreifen des Hygieneartikels gemäß Fig. 1 in jeweiliger Draufsicht,
- Fig. 5: in a) bis e) unterschiedliche Ausführungsbeispiele des absorbierenden Kerns und des Indikators mit Indikatorstreifen des Hygieneartikels gemäß Fig. 1 in jeweiliger Draufsicht,
- Fig. 6: in a) bis d) unterschiedliche Ausführungsbeispiele des absorbierenden Kerns und des Indikators mit Indikatorstreifen des Hygieneartikels gemäß Fig. 1 in jeweiliger Draufsicht und
- Fig. 7: in a) und b) sowie c) bis e) jeweils ein weiteres Ausführungsbeispiel des absorbierenden Kerns ohne bzw. mit Faltungsbahnen des Hygieneartikels gemäß Fig. 1 in Schnittdarstellung.

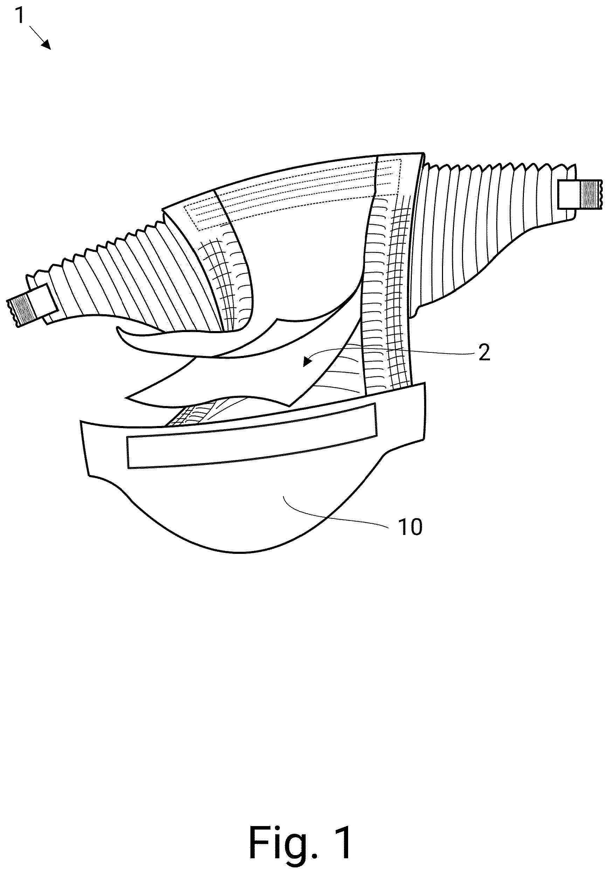

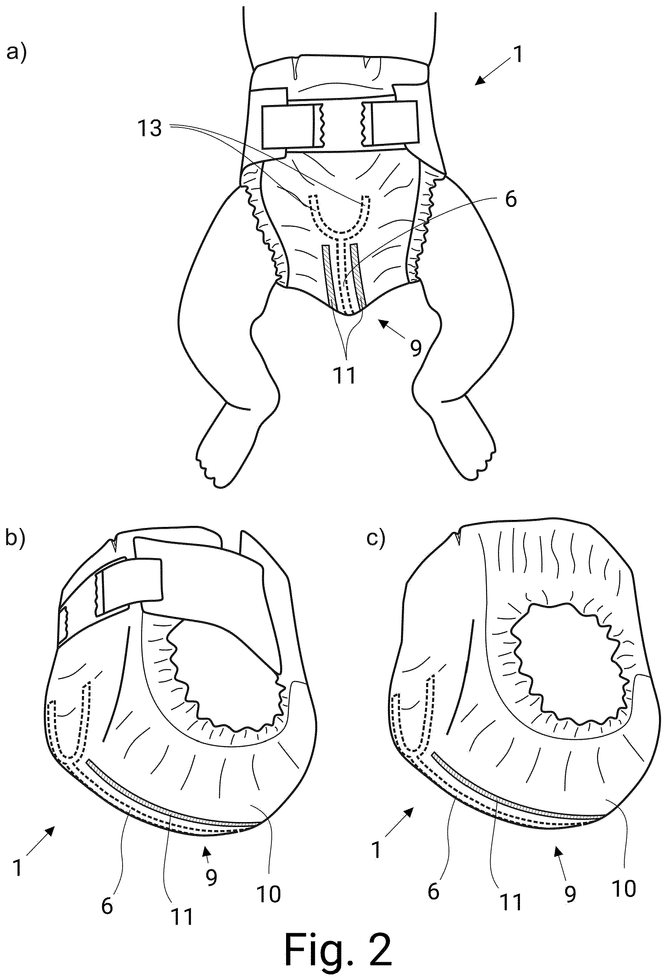

In Fig. 1 ist ein beispielhaft als Windel ausgebildeter Hygieneartikel 1 dargestellt. Hier und vorzugsweise ist die Windel eine Einwegwindel. Bezüglich weiterer bevorzugter Hygieneartikel 1 wird auf den einleitenden Teil der Beschreibung verwiesen. In Fig. 2a) ist der Hygieneartikel 1 in einem getragenen Zustand und in Fig. 2b) in einem ungetragenen Zustand abgebildet. Fig. 2c) zeigt eine alternative Ausführungsform des Hygieneartikels 1, wobei sich die Ausführungsformen ausschließlich hinsichtlich der Befestigungsmöglichkeit an einem Träger im getragenen Zustand unterscheiden.

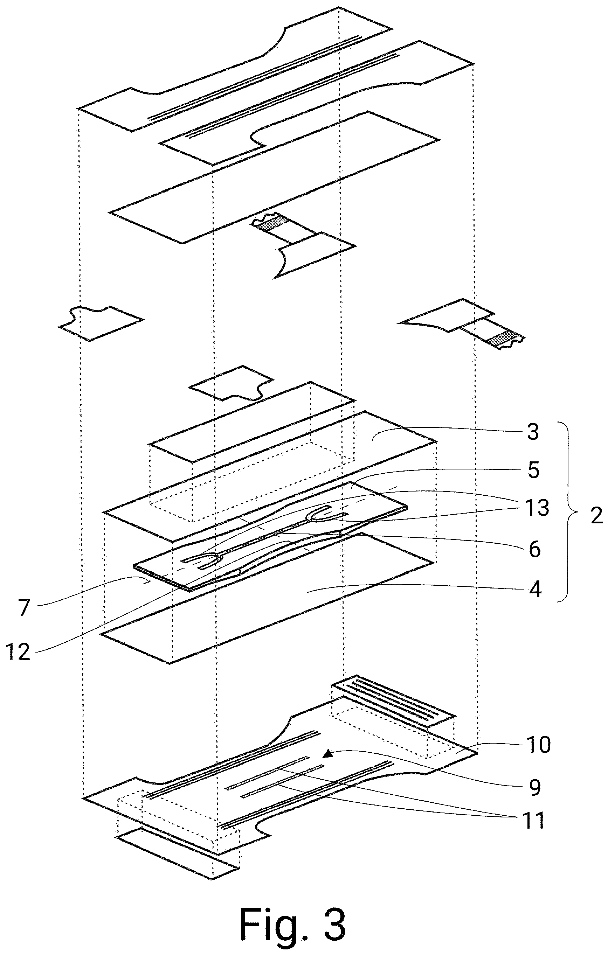

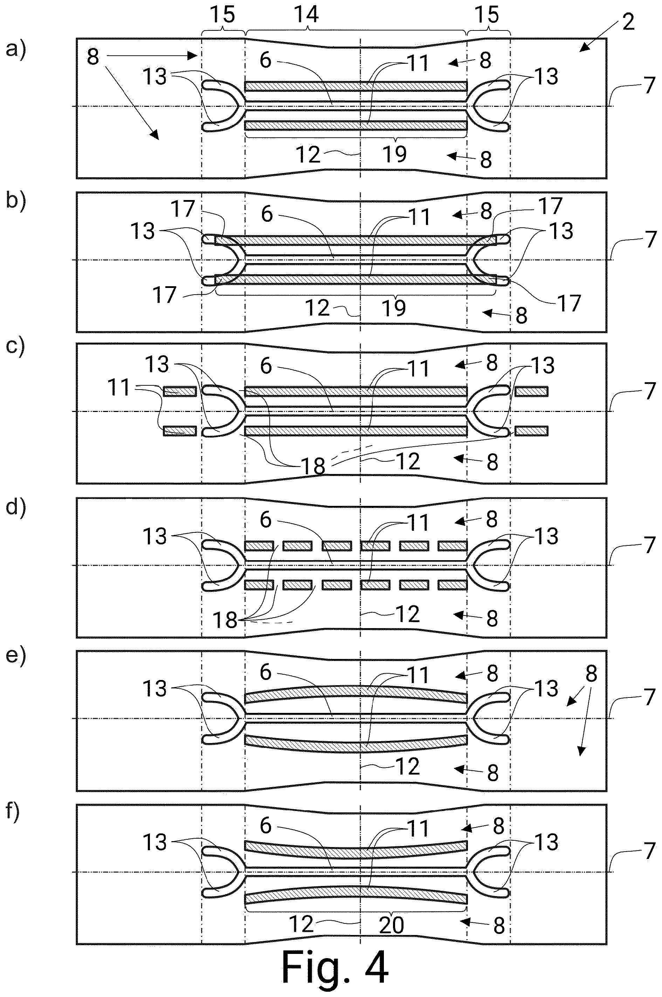

Der Hygieneartikel 1 weist einen absorbierenden Kern 2 zur Aufnahme von Flüssigkeit auf. Der absorbierende Kern 2 weist ein körperseitiges Deckblatt 3, ein körperfernes Rückenblatt 4 und eine zwischen dem Deckblatt 3 und dem Rückenblatt 4 angeordnete Schicht mit Saugmaterial 5 auf, wie es sich beispielsweise aus Fig. 3 ergibt. Die Schicht mit Saugmaterial 5 kann vorzugsweise Superabsorber und/oder Zellstoff umfassen. Das Deckblatt 3 kann vorzugsweise einen Vliesstoff (nonwoven) umfassen. Das Rückenblatt 4 kann vorzugsweise einen Vliesstoff (nonwoven) umfassen. In den Figuren 4a) bis 4f), 5a) bis 5e) und 6a) bis 6d) sind unterschiedliche Ausgestaltungsbeispiele des absorbierenden Kerns 2 dargestellt.

Der absorbierende Kern 2 weist einen Längskanal 6 auf, so wie es etwa aus den Figuren 2a) bis 2c) und 3 hervorgeht. Der Längskanal 6 verläuft zumindest abschnittsweise, insbesondere vollständig, mittig entlang der Längsachse 7 des absorbierenden Kerns 2. Hier und vorzugsweise ist der Längskanal 6, insbesondere ausschließlich, gerade ausgebildet. Gleichwohl ist es denkbar, dass der Längskanal 6 abschnittsweise einen kurvigen Verlauf aufweist. "Mittig entlang der Längsachse" bedeutet in diesem Zusammenhang, dass der Längskanal 6 mittig bezogen auf die Erstreckung des absorbierenden Kerns 2 in Querrichtung in Richtung der Längsachse 7 verläuft. Es ist möglich, dass der Längskanal 6, wie hier, vollständig, also ausschließlich, auf der Längsachse 7 angeordnet ist und damit ausschließlich mittig entlang der Längsachse 7 verläuft. Dies kann etwa der Fall sein, wenn der Längskanal 6 gerade ausgebildet ist. Alternativ ist es jedoch ebenso denkbar, dass der Längskanal 6 abschnittsweise auf der Längsachse 7 und abschnittsweise um die Längsachse 7 herum angeordnet ist und damit abschnittsweise mittig entlang der Längsachse 7 und abschnittsweise neben der Längsachse 7 verläuft. Dies kann etwa der Fall sein, wenn der Längskanal 6 abschnittsweise einen geraden Verlauf, welcher auf der Längsachse 7 angeordnet ist, und abschnittsweise einen kurvigen Verlauf aufweist, welcher zumindest teilweise nicht auf der Längsachse 7 angeordnet ist.

Der Längskanal 6 unterteilt die Schicht mit Saugmaterial 5 in zwei Saugabschnitte 8. Dies erfolgt derart, dass einer der Saugabschnitte 8 linksseitig und einer der Saugabschnitte 8 rechtsseitig gegenüber dem Längskanal 6 angeordnet ist. "Linksseitig" und "rechtsseitig" in diesem Zusammenhang beziehen sich auf den von vorne auf den Hygieneartikel 1 blickenden Betrachter im getragenen Zustand, so dass etwa in Fig. 2a) der rechtsseitige Saugabschnitt 8 rechts und der linksseitige Saugabschnitt 8 links abgebildet ist. Die Saugabschnitte 8 können durch den Längskanal 6 räumlich voneinander getrennt sein, wobei in den Saugabschnitten 8 insbesondere das Saugmaterial der Schicht mit Saugmaterial 5 räumlich voneinander getrennt sein kann. Die Saugabschnitte 8 erstrecken sich bezogen auf die Richtung der Längsachse 7 des absorbierenden Kerns 2, zumindest über die Länge des Längskanals 6, so wie es etwa aus Fig. 3 ersichtlich ist. Es ist möglich, dass sich die Saugabschnitte 8 in Richtung der Längsachse 7 über die Länge des Längskanals 6 hinaus erstrecken. Es ist möglich, dass die Saugabschnitte 8 in Längsabschnitten des absorbierenden Kerns 2 ohne Längskanal 6 durch die Längsachse 7 festgelegt sind. Eine räumliche Trennung der Saugabschnitte 8 zueinander ist in diesen Längsabschnitten ohne Längskanal 6 nicht erforderlich.

Der Hygieneartikel 1 weist ferner einen Indikator 9 zur Anzeige von durch den absorbierenden Kern 2 aufgenommener Flüssigkeit auf. In den Figuren 4a) bis 4f), 5a) bis 5e) und 6a) bis 6d) ist der Indikator 9 jeweils mit dargestellt, um die Lagebeziehungen zwischen den Komponenten des Hygieneartikels 1 zueinander zu verdeutlichen. Gleichwohl ist es nicht zwingend erforderlich, dass der Indikator 9 als Teil des absorbierenden Kerns 2 ausgebildet oder dass der Indikator 9 an dem absorbierenden Kern 2 angeordnet ist. Vielmehr ist es vorzugsweise so, dass der Indikator 9 an einem (nachfolgend noch beschriebenen) Backsheet 10 des Hygieneartikels 1 angeordnet ist. Der Indikator 9 kann mit dem absorbierenden Kern 2 in Kontakt sein.

Wesentlich ist nun, dass der Indikator 9 zwei Indikatorstreifen 11 umfasst und dass einer der Indikatorstreifen 11 linksseitig und einer der Indikatorstreifen 11 rechtsseitig von dem Längskanal 6 angeordnet und dem jeweiligen der Saugabschnitte 8 zugeordnet ist.

Der Längskanal 6 kann eine gute Flüssigkeitsverteilung in dem absorbierenden Kern 2 ermöglichen. Da der Längskanal 6 im Wesentlichen mittig entlang der Längsachse 7 verläuft, kann insbesondere in Richtung der Längsachse 7 die Flüssigkeitsverteilung durch den Längskanal 6 erfolgen. Die Flüssigkeit kann, insbesondere ausgehend von dem Längskanal 6, in die Saugabschnitte 8 verteilt werden, so dass etwa eine gleichmäßige Flüssigkeitsaufnahme erfolgen kann. Innerhalb der Saugabschnitte 8 selbst kann ebenfalls das Verteilen der Flüssigkeit, insbesondere in Richtung der Längsachse 7 und/oder der Querachse 12 des absorbierenden Kerns 2, erfolgen.

Durch die Anordnung der Indikatorstreifen 11 linksseitig und rechtsseitig von dem Längskanal 6 und insbesondere durch die Zuordnung der Indikatorstreifen 11 zu dem jeweiligen Saugabschnitt 8 kann eine verbesserte Anzeigezuverlässigkeit erreicht werden. Sobald beispielsweise einer oder beide der Saugabschnitte 8 eine bestimmte Menge an Flüssigkeit aufgenommen haben, kann der Indikatorstreifen 11 dies anzeigen und der Hygieneartikel 1 ggf. gewechselt werden.

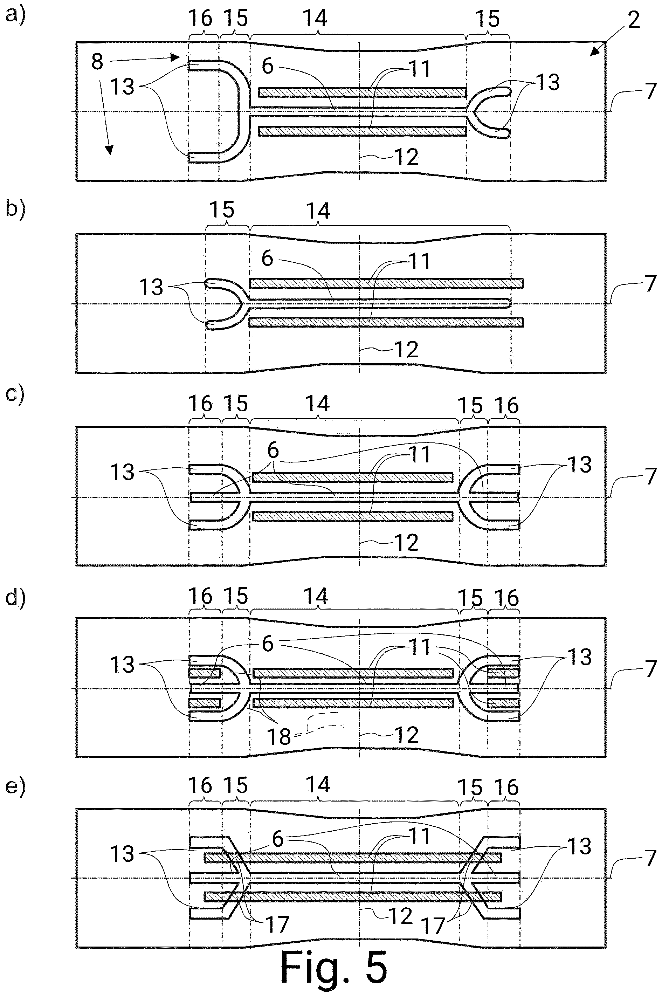

Ganz grundsätzlich hat es sich mit Blick auf die Flüssigkeitsverteilung als bevorzugt herausgestellt, wenn der absorbierende Kern 2 mehrere Verteilerkanäle 13 aufweist und wenn die Verteilerkanäle 13, vorzugsweise ausgehend von dem Längskanal 6, jeweils zumindest abschnittsweise in Richtung der Längsachse 7 des absorbierenden Kerns 2 und in Richtung der Querachse 12 des absorbierenden Kerns 2 verlaufen. Hierbei ist es grundsätzlich denkbar, dass die Verteilerkanäle 13 abschnittsweise sowohl in Richtung der Längsachse 7 als auch in Richtung der Querachse 12 und/oder ausschließlich in Richtung der Längsachse 7 oder ausschließlich in Richtung der Querachse 12 verlaufen. Die Verteilerkanäle 13 können jeweils zumindest abschnittsweise gerade und/oder gekrümmt ausgebildet sein. Vorteilhafte Ausgestaltungen diesbezüglich sind etwa den Figuren 5a) bis 5e) zu entnehmen.

Ganz allgemein kann der absorbierende Kern 2 einen mittleren Längsabschnitt 14 aufweisen. Der Längskanal 6 kann insbesondere in dem mittleren Längsabschnitt 14 angeordnet sein. Es ist möglich, dass die Verteilerkanäle 13, insbesondere in einem ersten Längsabschnitt 15 oder mehreren ersten Längsabschnitten 15 des absorbierenden Kerns 2, in Richtung der Längsachse 7 des absorbierenden Kerns 2 und in Richtung der Querachse 12 des absorbierenden Kerns 2 verlaufen, so wie es etwa für die linksseitig dargestellten Verteilerkanäle 13 bei dem Ausführungsbeispiel in Fig. 3 vorgesehen sowie in den Figuren 5a) bis 5e) abgebildet ist. Hierdurch kann sich, insbesondere in dem ersten Längsabschnitt 15 oder in den ersten Längsabschnitten 15, jeweils ein gekrümmter (bspw. Fig. 5b)) oder gerader Verlauf (bspw. Fig. 5e)) der Verteilerkanäle 13 ergeben. Der erste Längsabschnitt 15 oder die ersten Längsabschnitte 15 des absorbierenden Kerns 2 können an den mittleren Längsabschnitt 14 angrenzen und/oder bezogen auf die Richtung der Längsachse 7 neben dem mittleren Längsabschnitt 14 angeordnet sein. Der mittlere Längsabschnitt 14 ist insbesondere verteilerkanalfrei 13 ausgebildet. Der mittlere Längsabschnitt 14 ist damit insbesondere jener Längsabschnitt des absorbierenden Kerns 2, über welchen sich der Längskanal 6 erstreckt und in welchem keine Verteilerkanäle 13 angeordnet sind. Es ist möglich, dass sich der Längskanal 6 über den mittleren Längsabschnitt 14 hinaus, beispielsweise in den ersten Längsabschnitt 15 hinein, erstreckt, wie es etwa in den Figuren 5c) bis 5e) der Fall ist.

Mit Blick auf Fig. 5a) und vorzugsweise ist es denkbar, dass die Verteilerkanäle 13 abschnittsweise ausschließlich in Richtung der Querachse 12 des absorbierenden Kerns 2 verlaufen. Mit Blick auf die Figuren 5a), 5c) bis 5e) und vorzugsweise ist es denkbar, dass die Verteilerkanäle 13 zumindest abschnittsweise, insbesondere in einem zweiten Längsabschnitt 16 oder in mehreren zweiten Längsabschnitten 16 des absorbierenden Kerns 2, ausschließlich in Richtung der Längsachse 7 des absorbierenden Kerns 2 verlaufen. Der zweite Längsabschnitt 16 oder die zweiten Längsabschnitte 16 können an den ersten Längsabschnitt 15 oder jeweils an einen der ersten Längsabschnitte 15 angrenzen und/oder neben dem ersten Längsabschnitt 15 oder bezogen auf die Richtung der Längsachse 7 jeweils neben einem der ersten Längsabschnitte 15 angeordnet sein.

Um die Flüssigkeitsverteilung weiter zu optimieren und insbesondere hinsichtlich der Anatomie des Trägers anzupassen, ist vorzugsweise vorgesehen, dass die Verteilerkanäle 13 bezogen auf die Längsachse 7 und/oder die Richtung der Längsachse 7 des absorbierenden Kerns 2 vor und/oder hinter dem Längskanal 6 angeordnet sind. Die Verteilerkanäle 13 können also einseitig oder beidseitig neben dem Längskanal 6 angeordnet sein. Bei den Ausführungsbeispielen gemäß den Figuren 3, 4a) bis 4f), 5a) und 6a) bis 6d) sind die Verteilerkanäle 13 beispielhaft vor und hinter dem Längskanal 6 angeordnet, bei dem Ausführungsbeispiel gemäß Fig. 5b) sind die Verteilerkanäle 13 ausschließlich vor dem Längskanal 6 angeordnet.

Alternativ oder zusätzlich ist es denkbar, dass die Verteilerkanäle 13 von dem Längskanal 6 derart abgehend verlaufen, dass die Verteilerkanäle 13 und der Längskanal 6 zumindest abschnittsweise, hier und vorzugsweise in dem zweiten Längsabschnitt 16 oder den zweiten Längsabschnitten 16, nebeneinander, vorzugsweise parallel, insbesondere bezogen auf die Richtung der Querachse 12, angeordnet sind (Figuren 5c) bis 5e)).

Weitgehend unabhängig davon, wie nunmehr die Struktur des Hygieneartikels 1 und insbesondere des absorbierenden Kerns 2 hinsichtlich einer guten Flüssigkeitsverteilung ausgebildet ist, haben sich im Hinblick auf den Indikator 9 unterschiedliche Ausgestaltungen als vorteilhaft herausgestellt. So ist vorzugsweise vorgesehen, dass die Indikatorstreifen 11 und der Längskanal 6 und/oder die Indikatorstreifen 11 und die Verteilerkanäle 13 schnittpunktfrei zueinander angeordnet sind. Aufgrund der Tatsache, dass die Indikatorstreifen 11 und der Längskanal 6 und/oder die Verteilerkanäle 13 auf unterschiedlichen Schichten des Hygieneartikels 1 angeordnet sein können (etwa auf dem Backsheet 10 und dem absorbierenden Kern 2), meint "schnittpunktfrei" in diesem Sinne, dass eine gedachte Projektion der Indikatorstreifen 11 und des Längskanals 6 und/oder der Verteilerkanäle 13 auf eine gemeinsame Ebene, welche bspw. durch den absorbierenden Kern 2 verläuft (wie es aus den Figuren 4a) bis 4f), 5a) bis 5e) sowie 6a) bis 6d) ersichtlich ist), zu keinen entsprechenden Schnittpunkten führt.

Beispielsweise bei den Ausführungsbeispielen gemäß den Figuren 3, 4a), 4c) bis 4f), 5a) bis 5d) sowie 6a) bis 6d) sind die Indikatorstreifen 11 und der Längskanal 6 und die Verteilerkanäle 13 schnittpunktfrei zueinander angeordnet. Da insbesondere in dem Längskanal 6 und/oder in den Verteilerkanälen 13 vergleichsweise frühzeitig Flüssigkeit sein wird, kann die zumindest teilweise schnittpunktfreie Anordnung einer verfrühten Anzeige durch die Indikatorstreifen 11 entgegenwirken.

Es ist vorzugsweise möglich, dass die Indikatorstreifen 11 gegenüber dem Längskanal 6 jeweils in einem Indikatorabstand beabstandet angeordnet sind. Der Indikatorabstand entspricht dabei mindestens 5 % der minimalen Erstreckung des absorbierenden Kerns 2 in Richtung der Querachse 12, vorzugsweise mindestens 10 %, weiter vorzugsweise mindestens 20 %. Mit anderen Worten sind die Indikatorstreifen 11 jeweils von dem Längskanal 6 in Richtung der Querachse 12 in dem Indikatorabstand angeordnet, wobei der Indikatorabstand mindestens 5 %, vorzugsweise mindestens 10 %, weiter vorzugsweise mindestens 20%, von der minimalen Breite des absorbierenden Kerns 2 beträgt. Es ist alternativ oder zusätzlich möglich, dass die Indikatorstreifen 11 gegenüber dem Längskanal 6 jeweils in einem Indikatorabstand von höchstens 80 % der minimalen Erstreckung des absorbierenden Kerns 2 in Richtung der Querachse 12, vorzugsweise höchstens 60 %, weiter vorzugsweise höchstens 50 %, beabstandet angeordnet sind.

Alternativ oder zusätzlich ist in diesem Zusammenhang vorzugsweise vorgesehen, dass die Indikatorstreifen 11 und der Längskanal 6 und/oder die Indikatorstreifen 11 und die Verteilerkanäle 13 jeweils zumindest einen gemeinsamen Schnittpunkt 17 haben. Ebenso, wie bereits im Zusammenhang mit der Schnittpunktfreiheit erwähnt, meint, aufgrund der Tatsache, dass die Indikatorstreifen 11 und der Längskanal 6 und/oder die Verteilerkanäle 13 auf unterschiedlichen Schichten des Hygieneartikels 1 angeordnet sein können, "ein gemeinsamer Schnittpunkt" 17 in diesem Sinne, dass eine gedachte Projektion der Indikatorstreifen 11 und des Längskanals 6 und/oder der Verteilerkanäle 13 auf eine gemeinsame Ebene, welche bspw. durch den absorbierenden Kern 2 verläuft, zu zumindest einem entsprechenden Schnittpunkt führt.

Etwa bei den Ausführungsbeispielen gemäß den Figuren 4b) und 5e) weisen die Indikatorstreifen 11 und die Verteilerkanäle 13 jeweils zwei gemeinsame Schnittpunkte 17 auf. Etwa für Hygieneartikel 1, bei denen eine vergleichsweise frühzeitige Anzeige durch den Indikator 9 erreicht werden soll, kann dies vorteilhaft sein. Hier und vorzugsweise können die Indikatorstreifen 11 und die Verteilerkanäle 13 den gemeinsamen Schnittpunkt 17 oder die gemeinsamen Schnittpunkte 17 jeweils in dem ersten Längsabschnitt 15 oder in einem der ersten Längsabschnitte 15 des absorbierenden Kerns 2 aufweisen.

Weiter ist hier und vorzugsweise vorgesehen, dass die Indikatorstreifen 11 symmetrisch, insbesondere spiegelsymmetrisch, gegenüber der Längsachse 7 des absorbierenden Kerns 2 angeordnet sind. Dies kann insbesondere vorgesehen sein, wenn der absorbierende Kern 2 und/oder der Längskanal 6 und/oder die Verteilerkanäle 13 symmetrisch gegenüber der Längsachse 7 des absorbierenden Kerns 2 ausgestaltet ist bzw. sind, so wie es bei allen dargestellten Ausführungsbeispielen der Fall ist.

Hinsichtlich der Indikatorstreifen 11 ist hier und weiter vorzugsweise vorgesehen, dass die Indikatorstreifen 11 jeweils als zusammenhängender Indikatorstreifen 11 ausgebildet sind, oder, dass die Indikatorstreifen 11 jeweils als Indikatorstreifen 11 mit einer oder mehreren Unterbrechungsstellen 18 ausgebildet sind. "Zusammenhängender Indikatorstreifen" in diesem Zusammenhang umfasst insbesondere Indikatorstreifen 11, welche entlang deren Verlauf unterbrechungsfrei ausgebildet sind, so wie etwa in den Figuren 3, 4a), 4b), 4e), 4f), 5a) bis 5c), 5e) sowie 6a) bis 6d) dargestellt. "Unterbrechungsstelle" in diesem Zusammenhang bezeichnet insbesondere eine Stelle entlang des Verlaufs des entsprechenden Indikatorstreifens 11, an welcher kein Indikatormaterial vorhanden ist, so dass an dieser Stelle keine Anzeige von durch den absorbierenden Kern 2 aufgenommener Flüssigkeit möglich ist. Die Ausführungsbeispiele gemäß den Figuren 4c), 4d) und 5d) weisen jeweils mehrere entsprechende Unterbrechungsstellen 18 auf. Die Unterbrechungsstelle 18 kann oder die Unterbrechungsstellen 18 können jeweils mindestens eine Länge von 3 mm, vorzugsweise mindestens 5 mm, weiter vorzugsweise von mindestens 10 mm aufweisen. Die Unterbrechungsstelle 18 kann oder die Unterbrechungsstellen 18 können jeweils eine Länge von höchstens 40 mm, vorzugsweise höchstens 20 mm, weiter vorzugsweise von höchstens 15 mm aufweisen. Besonders bevorzugt ist die Länge der Unterbrechungsstelle 18 oder der Unterbrechungsstellen 18 jeweils zwischen 5 mm bis 25 mm.

Unabhängig davon, ob die Indikatorstreifen 11 unterbrechungsfrei oder mit einer oder mehreren Unterbrechungsstellen 18 ausgebildet sind, ist hier und vorzugsweise vorgesehen, dass die Indikatorstreifen 11 jeweils einen geraden Indikatorabschnitt 19 aufweisen. Es ist vorzugsweise denkbar, dass die Indikatorstreifen 11 jeweils ausschließlich den geraden Indikatorabschnitt 19 aufweisen, also vollständig gerade ausgebildet sind, so wie es bei den Ausführungsbeispielen der Figuren 4a) bis 4d), 5a) bis 5e) sowie 6a) bis 6d) der Fall ist. In diesem Zusammenhang ist der jeweilige gerade Indikatorabschnitt 19 vorzugsweise parallel gegenüber der Längsachse 7 des absorbierenden Kerns 2 angeordnet. Der jeweilige gerade Indikatorabschnitt 19 kann gegenüber der Längsachse 7 des absorbierenden Kerns 2 mindestens, insbesondere genau, den Indikatorabstand, also den Abstand des jeweiligen Indikatorstreifens 11 zu der Längsachse 7, aufweisen.

Alternativ oder zusätzlich weisen die Indikatorstreifen 11 jeweils einen gekrümmten Indikatorabschnitt 20 auf. Der jeweilige gekrümmte Indikatorabschnitt 20 ist hierbei vorzugsweise konkav oder konvex. "Konkav" und "konvex" sind hier insbesondere auf die Längsachse 7 des absorbierenden Kerns 2 bzw. den Längskanal 6 bezogen. Der jeweils konkav gekrümmte Indikatorabschnitt 20 ist insoweit zur Längsachse 7 bzw. dem Längskanal 6 hin gekrümmt (Fig. 4f)), der jeweils konvex gekrümmte Indikatorabschnitt 20 ist insoweit von der Längsachse 7 bzw. dem Längskanal 6 weg gekrümmt (Fig. 4e)). Es ist vorzugsweise denkbar, dass die Indikatorstreifen 11 jeweils ausschließlich den gekrümmten Indikatorabschnitt 20 aufweisen, also vollständig gekrümmt ausgebildet sind, so wie es bei den Ausführungsbeispielen der Figuren 4e) und 4f) der Fall ist.

Es ist in diesem Zusammenhang ganz allgemein möglich, dass die Indikatorstreifen 11 jeweils mehrere gerade Indikatorabschnitte 19 aufweisen, welche etwa durch einen entsprechend gekrümmten Indikatorabschnitt 20 miteinander verbunden sind. Ebenso ist es möglich, dass die Indikatorstreifen 11 jeweils mehrere gekrümmte Indikatorabschnitte 20 aufweisen, welche beispielsweise durch einen entsprechend geraden Indikatorabschnitt 19 miteinander verbunden sind.

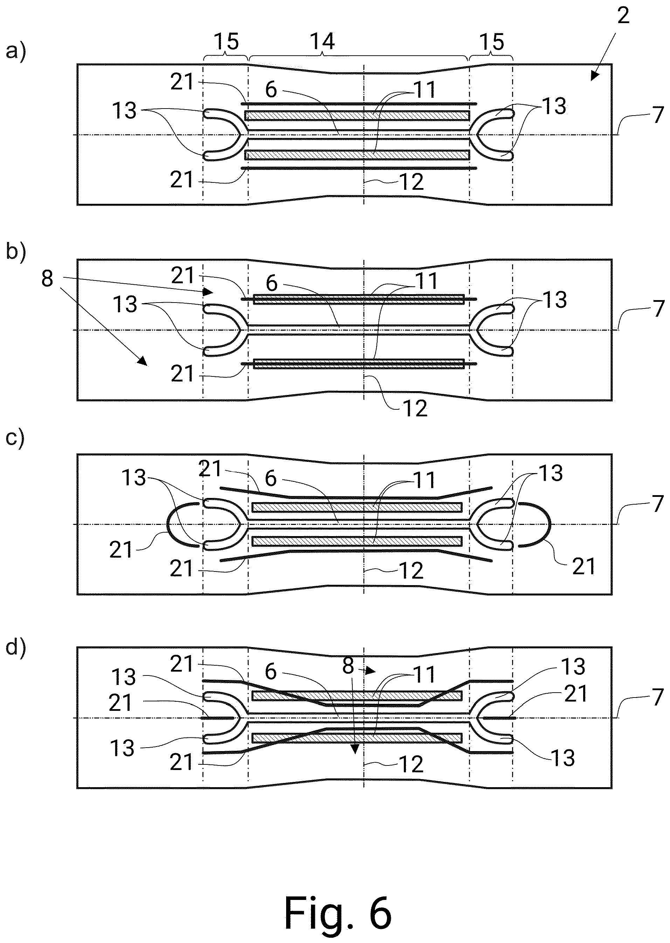

Die Ausgestaltung der Indikatorstreifen 11 hinsichtlich deren jeweiliger Länge kann ganz grundsätzlich auf unterschiedliche Art und Weise erfolgen. So ist es möglich, dass die Indikatorstreifen 11 sich über einen Teil des mittleren Längsabschnitts 14 (Figuren 5a), 5c), 5d) und 6b) bis 6d)) oder vollständig über den mittleren Längsabschnitt 14 (Figuren 4a) bis 4f), 5b), 5e), und Fig. 6a)) erstrecken. Es ist möglich, dass die Indikatorstreifen 11 ausschließlich in dem mittleren Längsabschnitt 14 (Figuren 4a), 4d) bis 4f), 5a), 5b) 5c) und 6b) bis 6d)) angeordnet sind. Es ist auch möglich, dass die Indikatorstreifen 11 zumindest abschnittsweise in dem ersten Längsabschnitt 15 oder in zumindest einem der ersten Längsabschnitte 15 (Figuren 4b), 5e) und 6a)) und/oder in dem zweiten Längsabschnitt 16 oder in zumindest einem der zweiten Längsabschnitte 16 (Figuren 4b), 4c), 5d), 5e)) angeordnet sind.

Es ist vorzugsweise vorgesehen, dass sich die Indikatorstreifen 11 entlang der Längsachse 7 des absorbierenden Kerns 2 über mindestens 20 %, vorzugsweise mindestens 30 %, weiter vorzugsweise mindestens 40 %, der Länge des absorbierenden Kerns 2 erstrecken. "Entlang der Längsachse" in diesem Zusammenhang bedeutet nicht, dass die Indikatorstreifen 11 mittig auf der Längsachse 7 angeordnet sind, sondern im Wesentlichen entlang dieser, also bspw. parallel zu dieser, verlaufen. Es ist denkbar, dass sich die Indikatorstreifen 11 entlang der Längsachse 7 des absorbierenden Kerns 2 über höchstens 80 %, vorzugsweise höchstens 70 %, weiter vorzugsweise höchstens 60 %, der Länge des absorbierenden Kerns 2 erstrecken. Alternativ oder zusätzlich ist vorzugsweise vorgesehen, dass sich die Indikatorstreifen 11 entlang der Längsachse 7 des absorbierenden Kerns 2 über mindestens 50 %, vorzugsweise mindestens 60 %, weiter vorzugsweise mindestens 70 %, der Länge des Längskanals 6 erstrecken. Es ist möglich, dass sich die Indikatorstreifen 11 über die gesamte Länge des Längskanals 6 und ggf. darüber hinaus erstreckt.

Ganz grundsätzlich ist es denkbar, die Indikatorstreifen 11 direkt an dem absorbierenden Kern 2 anzuordnen. Hierdurch lässt sich sicherstellen, dass die Indikatorstreifen 11 mit der von dem absorbierenden Kern 2 aufgenommenen Flüssigkeit in Kontakt gelangen und entsprechend zuverlässig anzeigen können. Gleichwohl ergibt sich hierbei der Nachteil, dass die Indikatorstreifen 11 in dem getragenen Zustand hierbei nicht oder nur eingeschränkt sichtbar sind. Es ist daher ganz besonders vorzugsweise vorgesehen, dass der Hygieneartikel 1 ein flüssigkeitsundurchlässiges Backsheet 10 aufweist und dass die Indikatorstreifen 11 an dem Backsheet 10, insbesondere an einer Innenseite des Backsheets 10, angeordnet sind. Das Backsheet 10 wurde vorstehend bereits in einem anderen Zusammenhang erwähnt. Die "Innenseite des Backsheets" bezeichnet jene Seite des Backsheets 10, welche dem absorbierten Kern 2 zugewandt ist. Die Indikatorstreifen 11 sind insbesondere derart an dem Backsheet 10 angeordnet, dass die Indikatorstreifen 11 mit dem absorbierenden Kern 2, insbesondere mit dem Rückenblatt 4, in Kontakt sind (Fig. 3) und/oder dass die Indikatorstreifen 11 im getragenen Zustand, insbesondere von außen, sichtbar sind (Figuren 2a) bis 2c)).

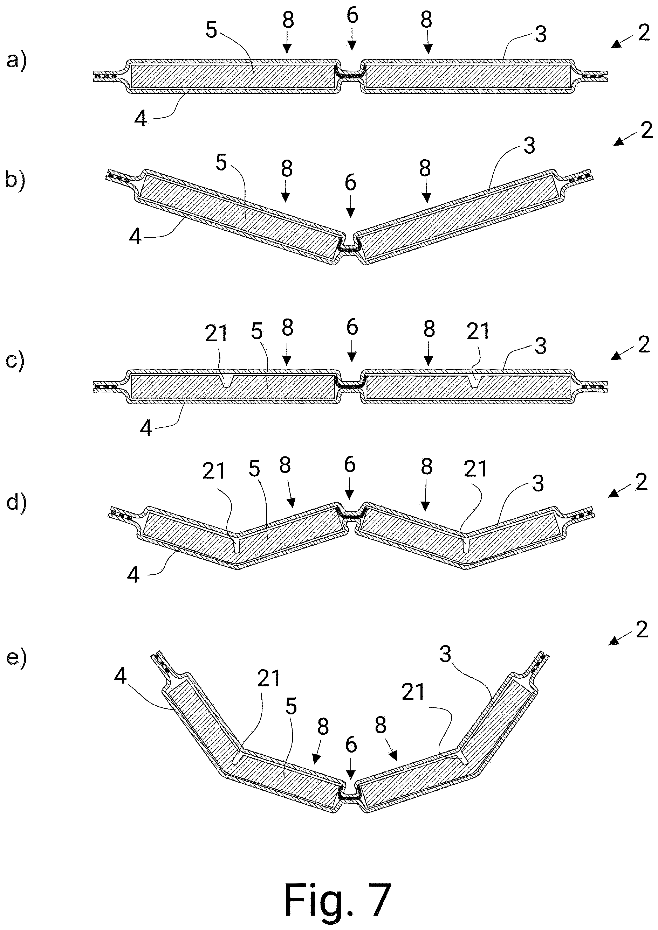

Die Figuren 7a), 7b) sowie 7c) bis 7e) zeigen jeweils eine Ausgestaltung des absorbierenden Kerns 2 in einem Querschnitt, bspw. durch die Längsmitte des absorbierenden Kerns 2. Der Längskanal 6 ist entsprechend quergeschnitten abgebildet. Mit Blick auf diese Figuren und vorzugsweise ist vorgesehen, dass das Deckblatt 3 und das Rückenblatt 4 im Bereich des Längskanals 6 miteinander verklebt und/oder gebondet sind. Der Längskanal 6 ist, hier und vorzugsweise, im Wesentlichen saugmaterialfrei. Der Längskanal 6 ist besonders vorzugsweise durch das Verkleben und/oder das Bonden des Deckblatts 3 und des Rückenblatts 4 ausgebildet. Der Längskanal 6 kann insoweit insbesondere einen Vliesstoff oder mehrere Vliesstoffe, vorzugsweise ausschließlich einen Vliesstoff oder mehrere Vliesstoffe, umfassen. Der Längskanal 6 kann vorzugsweise superabsorberfrei ausgebildet sein.

Weiterhin mit Blick auf die Figuren 7a), 7b) sowie 7c) bis 7e) ist ersichtlich, dass der absorbierende Kern 2 entlang des Längskanals 6 faltbar ist. Dies kann im Allgemeinen den Tragekomfort des Hygieneartikels 1 verbessern, wobei es sich im Zusammenhang mit dem Tragekomfort des Hygieneartikels 1 ganz besonders bewährt hat, wenn der absorbierende Kern 2, insbesondere die Schicht mit Saugmaterial 5, eine oder mehrere Faltungsbahnen 21 aufweist. Entlang der Faltungsbahn 21 oder den Faltungsbahnen 21 kann der absorbierende Kern 2 faltbar sein, so wie es etwa aus den Figuren 7d) und 7e) hervorgeht. Die Faltungsbahnen 21 können herstellungstechnisch in die Schicht mit Saugmaterial 5 geprägt sein. Die Figuren 6a) bis 6d) zeigen unterschiedlich ausgebildete Faltungsbahnen 21. Die Faltungsbahnen 21 sind hier und vorzugsweise symmetrisch, insbesondere spiegelsymmetrisch, gegenüber der Längsachse 7 des absorbierenden Kerns 2 angeordnet. Alternativ oder zusätzlich sind die Faltungsbahnen 21 zumindest abschnittsweise (Figuren 6c), 6d)), insbesondere zumindest in dem mittleren Längsabschnitt 14, oder ausschließlich (Figuren 6a), 6b)) parallel zueinander angeordnet.

Hinsichtlich der Anordnung der Indikatorstreifen 11 und der Faltungsbahnen 21 kann vorgesehen sein, dass die Indikatorstreifen 11 zumindest abschnittsweise, insbesondere in dem mittleren Längsabschnitt 14, in den Faltungsbahnen 21 verlaufen. Aufgrund der Tatsache, dass die Indikatorstreifen 11 und die Faltungsbahnen 21 auf unterschiedlichen Schichten des Hygieneartikels 1 angeordnet sein können, meint die zumindest abschnittsweise Anordnung der Indikatorstreifen 11 in den Faltungsbahnen 21 in diesem Sinne, dass in einer gedachten Projektion der Indikatorstreifen 11 und der Faltungsbahnen 21 auf eine gemeinsame Ebene, welche bspw. durch den absorbierenden Kern 2 verläuft (wie es aus den Figuren 6a) bis 6d) ersichtlich ist), die Indikatorstreifen 11 zumindest abschnittsweise in den Faltungsbahnen 21 verlaufen. Dies ist beispielsweise bei den Ausführungsbeispielen gemäß den Figuren 6b) und 6d) der Fall. Alternativ ist es auch möglich, dass die Indikatorstreifen 11 neben den Faltungsbahnen 21 verlaufen, wie es beispielsweise bei den Ausführungsbeispielen gemäß den Figuren 6a) und 6c) der Fall ist. Ganz allgemein können die Indikatorstreifen 11 bezogen auf die Richtung der Querachse 12 zwischen dem Längskanal 6 und Faltungsbahnen 21 angeordnet sein.

Vorgeschlagen wird gemäß einer weiteren Lehre ein Verfahren zur Herstellung eines Hygieneartikels 1, insbesondere einer Windel. Der Hygieneartikel 1 kann vorschlagsgemäß ausgebildet sein und eines oder mehrere der im Zusammenhang mit dem vorschlagsgemäßen Hygieneartikel 1 beschriebenen Merkmale aufweisen. Der Hygieneartikel 1, welcher durch das vorschlagsgemäße Verfahren herstellbar ist oder hergestellt wird, weist einen absorbierenden Kern 2 zur Aufnahme von Flüssigkeit und einen Indikator 9 zur Anzeige von durch den absorbierenden Kern 2 aufgenommener Flüssigkeit auf, wobei der absorbierende Kern 2 ein körperseitiges Deckblatt 3, ein körperfernes Rückenblatt 4 und eine zwischen dem Deckblatt 3 und dem Rückenblatt 4 angeordnete Schicht mit Saugmaterial 5, vorzugsweise umfassend Superabsorber, aufweist, wobei der absorbierende Kern 2 einen Längskanal 6 aufweist, wobei der Längskanal 6 zumindest abschnittsweise mittig entlang der Längsachse 7 des absorbierenden Kerns 2 verläuft, wobei der Längskanal 6 die Schicht mit Saugmaterial 5 derart in zwei Saugabschnitte 8 unterteilt, dass einer der Saugabschnitte 8 linksseitig und einer der Saugabschnitte 8 rechtsseitig gegenüber dem Längskanal 6 angeordnet ist. Wesentlich nach dieser weiteren Lehre ist, dass der Indikator 9 zwei Indikatorstreifen 11 umfasst und dass einer der Indikatorstreifen 11 linksseitig und einer der Indikatorstreifen 11 rechtsseitig von dem Längskanal 6 angeordnet und dem jeweiligen der Saugabschnitte 8 zugeordnet wird.

Auf alle Ausführungen zu dem vorschlagsgemäßen Hygieneartikel 1 darf verwiesen werden.

Es ist im Zusammenhang mit dem Verfahren denkbar, dass die Indikatorstreifen 11 aufgetragen, insbesondere aufgesprüht, werden. Dies kann nacheinander oder zumindest teilweise zeitgleich erfolgen. Mit Blick auf Fig. 3 und vorzugsweise ist vorgesehen, dass die Indikatorstreifen 11 auf einer Innenseite des Backsheets 10 angeordnet, insbesondere aufgetragen, werden. Das Backsheet 10 kann zumindest teilweise derart transparent ausgebildet sein, dass die Indikatorstreifen 11 im getragenen Zustand des Hygieneartikels 1 sichtbar sind. In diesem Fall kann das Anordnen der Indikatorstreifen 11 links- und rechtsseitig von dem Längskanal 6 und das Zuordnen zu dem jeweiligen der Saugabschnitte 8 beispielsweise beim Anordnen des absorbierenden Kerns 2 an dem Backsheet 10 erfolgen. Es ist alternativ auch möglich, dass die Indikatorstreifen 11 auf den absorbierenden Kern 2, insbesondere auf das Rückenblatt 4 angeordnet, insbesondere aufgetragen, werden.

### Bezugszeichenliste

- 1: Hygieneartikel
- 2: absorbierender Kern
- 3: Deckblatt
- 4: Rückenblatt
- 5: Schicht mit Saugmaterial
- 6: Längskanal
- 7: Längsachse
- 8: Saugabschnitt
- 9: Indikator
- 10: Backsheet
- 11: Indikatorstreifen
- 12: Querachse
- 13: Verteilerkanal
- 14: mittlerer Längsabschnitt
- 15: erster Längsabschnitt
- 16: zweiter Längsabschnitt
- 17: gemeinsamer Schnittpunkt
- 18: Unterbrechungsstelle
- 19: gerader Indikatorabschnitt
- 20: gekrümmter Indikatorabschnitt
- 21: Faltungsbahn

## Patentansprüche

1. Hygieneartikel, insbesondere eine Windel, wobei der Hygieneartikel (1) einen absorbierenden Kern (2) zur Aufnahme von Flüssigkeit und einen Indikator (9) zur Anzeige von durch den absorbierenden Kern (2) aufgenommener Flüssigkeit aufweist, wobei der absorbierende Kern (2) ein körperseitiges Deckblatt (3), ein körperfernes Rückenblatt (4) und eine zwischen dem Deckblatt (3) und dem Rückenblatt (4) angeordnete Schicht mit Saugmaterial (5), vorzugsweise umfassend Superabsorber, aufweist, wobei der absorbierende Kern (2) einen Längskanal (6) aufweist, wobei der Längskanal (6) zumindest abschnittsweise mittig entlang der Längsachse (7) des absorbierenden Kerns (2) verläuft, wobei der Längskanal (6) die Schicht mit Saugmaterial (5) derart in zwei Saugabschnitte (8) unterteilt, dass einer der Saugabschnitte (8) linksseitig und einer der Saugabschnitte (8) rechtsseitig gegenüber dem Längskanal (6) angeordnet ist,
**dadurch gekennzeichnet,**
**dass** der Indikator (9) zwei Indikatorstreifen (11) umfasst und dass einer der Indikatorstreifen (11) linksseitig und einer der Indikatorstreifen (11) rechtsseitig von dem Längskanal (6) angeordnet und dem jeweiligen der Saugabschnitte (8) zugeordnet ist.

2. Hygieneartikel nach Anspruch 1, **dadurch gekennzeichnet, dass** der absorbierende Kern (2) mehrere Verteilerkanäle (13) aufweist und dass die Verteilerkanäle (13), vorzugsweise ausgehend von dem Längskanal (6), jeweils zumindest abschnittsweise in Richtung der Längsachse (7) des absorbierenden Kerns (2) und/oder in Richtung der Querachse (12) des absorbierenden Kerns (2) verlaufen.

3. Hygieneartikel nach Anspruch 1 oder 2, **dadurch gekennzeichnet, dass** die Verteilerkanäle (13) bezogen auf die Längsachse (7) des absorbierenden Kerns (2) vor und/oder hinter dem Längskanal (6) angeordnet sind, und/oder, dass die Verteilerkanäle (13) von dem Längskanal (6) derart abgehend verlaufen, dass die Verteilerkanäle (13) und der Längskanal (6) zumindest abschnittsweise, insbesondere in einem Längsabschnitt des absorbierenden Kerns (2), nebeneinander, vorzugsweise parallel, angeordnet sind.

4. Hygieneartikel nach einem der vorhergehenden Ansprüche, **dadurch gekennzeichnet, dass** die Indikatorstreifen (11) und der Längskanal (6) und/oder die Indikatorstreifen (11) und die Verteilerkanäle (13) schnittpunktfrei zueinander angeordnet sind.

5. Hygieneartikel nach einem der vorhergehenden Ansprüche, **dadurch gekennzeichnet, dass** die Indikatorstreifen (11) und der Längskanal (6) und/oder die Indikatorstreifen (11) und die Verteilerkanäle (13) jeweils zumindest einen gemeinsamen Schnittpunkt (17) haben.

6. Hygieneartikel nach einem der vorhergehenden Ansprüche, **dadurch gekennzeichnet, dass** die Indikatorstreifen (11) symmetrisch gegenüber der Längsachse (7) des absorbierenden Kerns (2) angeordnet sind.

7. Hygieneartikel nach einem der vorhergehenden Ansprüche, **dadurch gekennzeichnet, dass** die Indikatorstreifen (11) jeweils als zusammenhängender Indikatorstreifen (11) ausgebildet sind, oder, dass die Indikatorstreifen (11) jeweils als Indikatorstreifen (11) mit einer oder mehreren Unterbrechungsstellen (18) ausgebildet sind.

8. Hygieneartikel nach einem der vorhergehenden Ansprüche, **dadurch gekennzeichnet, dass** die Indikatorstreifen (11) jeweils, insbesondere ausschließlich, einen geraden Indikatorabschnitt (19) aufweisen, vorzugsweise, dass der jeweilige gerade Indikatorabschnitt (19) parallel gegenüber der Längsachse (7) des absorbierenden Kerns (2) angeordnet ist, und/oder, dass die Indikatorstreifen (11) jeweils, insbesondere ausschließlich, einen gekrümmten Indikatorabschnitt (20) aufweisen, vorzugsweise, dass der jeweilige gekrümmte Indikatorabschnitt (20) konkav oder konvex ist.

9. Hygieneartikel nach einem der vorhergehenden Ansprüche, **dadurch gekennzeichnet, dass** sich die Indikatorstreifen (11) entlang der Längsachse (7) des absorbierenden Kerns (2) über mindestens 20 % und/oder höchstens 80 % der Länge des absorbierenden Kerns (2) erstrecken, und/oder, dass sich die Indikatorstreifen (11) entlang der Längsachse (7) des absorbierenden Kerns (2) über mindestens 50 % der Länge des Längskanals (6) erstrecken.

10. Hygieneartikel nach einem der vorhergehenden Ansprüche, **dadurch gekennzeichnet, dass** der Hygieneartikel (1) ein flüssigkeitsundurchlässiges Backsheet (10) aufweist und dass die Indikatorstreifen (11) an dem Backsheet (10) angeordnet sind.

11. Hygieneartikel nach einem der vorhergehenden Ansprüche, **dadurch gekennzeichnet, dass** das Deckblatt (3) und das Rückenblatt (4) im Bereich des Längskanals (6) miteinander verklebt und/oder gebondet sind, vorzugsweise, dass der Längskanal (6) durch das Verkleben und/oder das Bonden des Deckblatts (3) und des Rückenblatts (4) ausgebildet ist.

12. Hygieneartikel nach einem der vorhergehenden Ansprüche, **dadurch gekennzeichnet, dass** der absorbierende Kern (2), insbesondere die Schicht mit Saugmaterial (5), eine oder mehrere Faltungsbahnen (21) aufweist, vorzugsweise, dass die Faltungsbahnen (21) symmetrisch gegenüber der Längsachse (7) des absorbierenden Kerns (2) angeordnet sind, und/oder, dass die Faltungsbahnen (21) zumindest abschnittsweise parallel zueinander angeordnet sind.

13. Hygieneartikel nach einem der vorhergehenden Ansprüche, **dadurch gekennzeichnet, dass** die Indikatorstreifen (11) zumindest abschnittsweise in den Faltungsbahnen (21) verlaufen.

14. Verfahren zur Herstellung eines Hygieneartikels (1), insbesondere einer Windel, nach einem der vorhergehenden Ansprüche, mit einem absorbierenden Kern (2) zur Aufnahme von Flüssigkeit und einem Indikator (9) zur Anzeige von durch den absorbierenden Kern (2) aufgenommener Flüssigkeit, wobei der absorbierende Kern (2) ein körperseitiges Deckblatt (3), ein körperfernes Rückenblatt (4) und eine zwischen dem Deckblatt (3) und dem Rückenblatt (4) angeordnete Schicht mit Saugmaterial (5), vorzugsweise umfassend Superabsorber, aufweist, wobei der absorbierende Kern (2) einen Längskanal (6) aufweist, wobei der Längskanal (6) zumindest abschnittsweise mittig entlang der Längsachse (7) des absorbierenden Kerns (2) verläuft, wobei der Längskanal (6) die Schicht mit Saugmaterial (5) derart in zwei Saugabschnitte (8) unterteilt, dass einer der Saugabschnitte (8) linksseitig und einer der Saugabschnitte (8) rechtsseitig gegenüber dem Längskanal (6) angeordnet ist,
**dadurch gekennzeichnet,**
**dass** der Indikator (9) zwei Indikatorstreifen (11) umfasst und dass einer der Indikatorstreifen (11) linksseitig und einer der Indikatorstreifen (11) rechtsseitig von dem Längskanal (6) angeordnet und dem jeweiligen der Saugabschnitte (8) zugeordnet wird.

## Claims

1. Hygiene article, in particular a nappy, wherein the hygiene article (1) has an absorbent core (2) for absorbing liquid and an indicator (9) for indicating liquid absorbed by the absorbent core (2), wherein the absorbent core (2) has a body-side cover sheet (3), a body-remote backsheet (4) and a layer arranged between the cover sheet (3) and the backsheet (4) and containing absorbent material (5), preferably comprising superabsorbers, wherein the absorbent core (2) has a longitudinal channel (6), wherein the longitudinal channel (6) runs at least partly centrally along the longitudinal axis (7) of the absorbent core (2), wherein the longitudinal channel (6) divides the layer containing absorbent material (5) into two absorption sections (8) in such a way that one of the absorption sections (8) is arranged on the left-hand side and one of the absorption sections (8) is arranged on the right-hand side in relation to the longitudinal channel (6),
**characterized**
**in that** the indicator (9) comprises two indicator strips (11), and in that one of the indicator strips (11) is arranged on the left-hand side and one of the indicator strips (11) is arranged on the right-hand side of the longitudinal channel (6) and assigned to one each of the absorption sections (8).

2. Hygiene article according to Claim 1, **characterized in that** the absorbent core (2) has a plurality of distributor channels (13), and **in that** the distributor channels (13), preferably proceeding from the longitudinal channel (6), each run at least partly in the direction of the longitudinal axis (7) of the absorbent core (2) and/or in the direction of the transverse axis (12) of the absorbent core (2).

3. Hygiene article according to Claim 1 or 2, **characterized in that** the distributor channels (13) are arranged in front of and/or behind the longitudinal channel (6) in relation to the longitudinal axis (7) of the absorbent core (2), and/or **in that** the distributor channels (13) run proceeding from the longitudinal channel (6) in such a way that the distributor channels (13) and the longitudinal channel (6) are at least partly arranged next to one another, preferably in parallel, in particular in a longitudinal section of the absorbent core (2).

4. Hygiene article according to one of the preceding claims, **characterized in that** the indicator strips (11) and the longitudinal channel (6) and/or the indicator strips (11) and the distributor channels (13) are arranged with respect to one another without any intersecting points.

5. Hygiene article according to one of the preceding claims, **characterized in that** the indicator strips (11) and the longitudinal channel (6) and/or the indicator strips (11) and the distributor channels (13) each have at least one common intersecting point (17).

6. Hygiene article according to one of the preceding claims, **characterized in that** the indicator strips (11) are arranged symmetrically with respect to the longitudinal axis (7) of the absorbent core (2).

7. Hygiene article according to one of the preceding claims, **characterized in that** the indicator strips (11) are each designed as a continuous indicator strip (11), or **in that** the indicator strips (11) are each designed as indicator strips (11) having one or more interruption points (18).

8. Hygiene article according to one of the preceding claims, **characterized in that** the indicator strips (11) each have, in particular exclusively, a straight indicator section (19), preferably **in that** the respective straight indicator section (19) is arranged parallel in relation to the longitudinal axis (7) of the absorbent core (2), and/or **in that** the indicator strips (11) each have, in particular exclusively, a curved indicator section (20), preferably **in that** the respective curved indicator section (20) is concave or convex.

9. Hygiene article according to one of the preceding claims, **characterized in that** the indicator strips (11) extend along the longitudinal axis (7) of the absorbent core (2) over at least 20% and/or at most 80% of the length of the absorbent core (2), and/or **in that** the indicator strips (11) extend along the longitudinal axis (7) of the absorbent core (2) over at least 50% of the length of the longitudinal channel (6).

10. Hygiene article according to one of the preceding claims, **characterized in that** the hygiene article (1) has a liquid-impermeable backsheet (10), and **in that** the indicator strips (11) are arranged on the backsheet (10).

11. Hygiene article according to one of the preceding claims, **characterized in that** the cover sheet (3) and the backsheet (4) are adhesively bonded and/or bonded to one another in the region of the longitudinal channel (6), preferably **in that** the longitudinal channel (6) is formed by the adhesive bonding and/or bonding of the cover sheet (3) and the backsheet (4).

12. Hygiene article according to one of the preceding claims, **characterized in that** the absorbent core (2), in particular the layer containing absorbent material (5), has one or more folding tracks (21), preferably **in that** the folding tracks (21) are arranged symmetrically with respect to the longitudinal axis (7) of the absorbent core (2), and/or **in that** the folding tracks (21) are arranged at least partly parallel to one another.

13. Hygiene article according to one of the preceding claims, **characterized in that** the indicator strips (11) run at least partly in the folding tracks (21).

14. Method for producing a hygiene article (1), in particular a nappy, according to one of the preceding claims, having an absorbent core (2) for absorbing liquid and an indicator (9) for indicating liquid absorbed by the absorbent core (2), wherein the absorbent core (2) has a body-side cover sheet (3), a body-remote backsheet (4) and a layer arranged between the cover sheet (3) and the backsheet (4) and containing absorbent material (5), preferably comprising superabsorbers, wherein the absorbent core (2) has a longitudinal channel (6), wherein the longitudinal channel (6) runs at least partly centrally along the longitudinal axis (7) of the absorbent core (2), wherein the longitudinal channel (6) divides the layer containing absorbent material (5) into two absorption sections (8) in such a way that one of the absorption sections (8) is arranged on the left-hand side and one of the absorption sections (8) is arranged on the right-hand side in relation to the longitudinal channel (6),
**characterized**
**in that** the indicator (9) comprises two indicator strips (11), and in that one of the indicator strips (11) is arranged on the left-hand side and one of the indicator strips (11) is arranged on the right-hand side of the longitudinal channel (6) and assigned to one each of the absorption sections (8).

## Revendications

1. Article d'hygiène, en particulier une couche-culotte, l'article d'hygiène (1) présentant une partie centrale absorbante (2) pour absorber un fluide et un indicateur (9) pour indiquer le fluide absorbé par la partie centrale absorbante (2), dans lequel la partie centrale absorbante (2) présente une feuille de recouvrement (3) côté corps, une feuille de fond (4) éloignée du corps et une couche disposée entre la feuille de recouvrement (3) et la feuille de fond (4) et comportant une matière absorbante (5), de préférence comprenant un superabsorbeur, dans lequel la partie centrale absorbante (2) présente un canal longitudinal (6), dans lequel le canal longitudinal (6) s'étend au moins par sections de manière centrale le long de l'axe longitudinal (7) de la partie centrale absorbante (2), dans lequel le canal longitudinal (6) divise la couche comportant une matière absorbante (5) en deux sections absorbantes (8) de telle sorte que l'une des sections absorbantes (8) est disposée du côté gauche et l'une des sections absorbantes (8) est disposée du côté droit par rapport au canal longitudinal (6),
**caractérisé en ce que**
l'indicateur (9) comprend deux bandes indicatrices (11) et **en ce que** l'une des bandes indicatrices (11) est disposée du côté gauche et l'une des bandes indicatrices (11) est disposée du côté droit du canal longitudinal (6) et est associée à la section respective parmi les sections absorbantes (8).

2. Article d'hygiène selon la revendication 1, **caractérisé en ce que** la partie centrale absorbante (2) présente plusieurs canaux de distribution (13) et **en ce que** les canaux de distribution (13) s'étendent, de préférence à partir du canal longitudinal (6), respectivement au moins par sections dans la direction de l'axe longitudinal (7) de la partie centrale absorbante (2) et/ou dans la direction de l'axe transversal (12) de la partie centrale absorbante (2).

3. Article d'hygiène selon la revendication 1 ou 2, **caractérisé en ce que** les canaux de distribution (13) sont disposés, par rapport à l'axe longitudinal (7) de la partie centrale absorbante (2), devant et/ou derrière le canal longitudinal (6), et/ou **en ce que** les canaux de distribution (13) s'étendent à partir du canal longitudinal (6) de telle sorte que les canaux de distribution (13) et le canal longitudinal (6) sont disposés côte à côte, de préférence parallèlement, au moins par sections, notamment dans une section longitudinale de la partie centrale absorbante (2).

4. Article d'hygiène selon l'une quelconque des revendications précédentes, **caractérisé en ce que** les bandes indicatrices (11) et le canal longitudinal (6) et/ou les bandes indicatrices (11) et les canaux de distribution (13) sont disposés sans point d'intersection les uns par rapport aux autres.

5. Article d'hygiène selon l'une quelconque des revendications précédentes, **caractérisé en ce que** les bandes indicatrices (11) et le canal longitudinal (6) et/ou les bandes indicatrices (11) et les canaux de distribution (13) présentent respectivement au moins un point d'intersection commun (17).

6. Article d'hygiène selon l'une quelconque des revendications précédentes, **caractérisé en ce que** les bandes indicatrices (11) sont disposées symétriquement par rapport à l'axe longitudinal (7) de la partie centrale absorbante (2).

7. Article d'hygiène selon l'une quelconque des revendications précédentes, **caractérisé en ce que** les bandes indicatrices (11) sont réalisées respectivement sous la forme de bandes indicatrices continues (11), ou **en ce que** les bandes indicatrices (11) sont réalisées respectivement sous la forme de bandes indicatrices (11) comportant un ou plusieurs points d'interruption (18).

8. Article d'hygiène selon l'une quelconque des revendications précédentes, **caractérisé en ce que** les bandes indicatrices (11) présentent respectivement, en particulier exclusivement, une section indicatrice droite (19), de préférence **en ce que** la section indicatrice droite respective (19) est disposée parallèlement à l'axe longitudinal (7) de la partie centrale absorbante (2), et/ou **en ce que** les bandes indicatrices (11) présentent respectivement, en particulier exclusivement, une section indicatrice courbe (20), de préférence **en ce que** la section indicatrice courbe respective (20) est concave ou convexe.

9. Article d'hygiène selon l'une quelconque des revendications précédentes, **caractérisé en ce que** les bandes indicatrices (11) s'étendent le long de l'axe longitudinal (7) de la partie centrale absorbante (2) sur au moins 20 % et/ou au plus 80 % de la longueur de la partie centrale absorbante (2), et/ou **en ce que** les bandes indicatrices (11) s'étendent le long de l'axe longitudinal (7) de la partie centrale absorbante (2) sur au moins 50 % de la longueur du canal longitudinal (6).

10. Article d'hygiène selon l'une quelconque des revendications précédentes, **caractérisé en ce que** l'article d'hygiène (1) présente une feuille arrière (10) imperméable aux fluides et **en ce que** les bandes indicatrices (11) sont disposées au niveau de la feuille arrière (10).

11. Article d'hygiène selon l'une quelconque des revendications précédentes, **caractérisé en ce que** la feuille de recouvrement (3) et la feuille de fond (4) sont collées et/ou liées l'une à l'autre dans la zone du canal longitudinal (6), de préférence **en ce que** le canal longitudinal (6) est formé par collage et/ou liaison de la feuille de recouvrement (3) et de la feuille de fond (4).

12. Article d'hygiène selon l'une quelconque des revendications précédentes, **caractérisé en ce que** la partie centrale absorbante (2), en particulier la couche présentant une matière absorbante (5), présente une ou plusieurs voies de pliage (21), de préférence **en ce que** les voies de pliage (21) sont disposées symétriquement par rapport à l'axe longitudinal (7) de la partie centrale absorbante (2), et/ou **en ce que** les voies de pliage (21) sont disposées parallèlement les unes aux autres au moins par sections.

13. Article d'hygiène selon l'une quelconque des revendications précédentes, **caractérisé en ce que** les bandes indicatrices (11) s'étendent au moins par sections dans les voies de pliage (21).

14. Procédé de fabrication d'un article d'hygiène (1), en particulier d'une couche-culotte, selon l'une quelconque des revendications précédentes, comportant une partie centrale absorbante (2) pour absorber un fluide et un indicateur (9) pour indiquer le fluide absorbé par la partie centrale absorbante (2), dans lequel la partie centrale absorbante (2) présente une feuille de recouvrement (3) côté corps, une feuille de fond (4) éloignée du corps et une couche disposée entre la feuille de recouvrement (3) et la feuille de fond (4) et comportant une matière absorbante (5), de préférence comprenant un superabsorbeur, dans lequel la partie centrale absorbante (2) présente un canal longitudinal (6), dans lequel le canal longitudinal (6) s'étend au moins par sections de manière centrale le long de l'axe longitudinal (7) de la partie centrale absorbante (2), dans lequel le canal longitudinal (6) divise la couche comportant une matière absorbante (5) en deux sections absorbantes (8) de telle sorte que l'une des sections absorbantes (8) est disposée du côté gauche et l'une des sections absorbantes (8) est disposée du côté droit par rapport au canal longitudinal (6),
**caractérisé en ce que**
l'indicateur (9) comprend deux bandes indicatrices (11) et **en ce que** l'une des bandes indicatrices (11) est disposée du côté gauche et l'une des bandes indicatrices (11) est disposée du côté droit du canal longitudinal (6) et est associée à la section respective parmi les sections absorbantes (8).
